# EUROPEAN PATENT SPECIFICATION

(11) **EP 4 260 869 B1**
(45) Date of publication and mention of the grant of the patent: **30.09.2026**
(21) Application number: 22167623.2
(22) Date of filing: 11.04.2022
(51) Int. Cl.: A61K 38/40, A61K 31/731, A61P 15/02, A61P 31/04, A61P 31/10, A61P 31/20, A61P 31/22, A61K 45/06

(54) **COMPOSITION COMPRISING A COMBINATION OF LACTOFERRIN AND A SULPHATED POLYSACCHARIDE**
ZUSAMMENSETZUNG MIT EINER KOMBINATION VON LACTOFERRIN UND EINEM SULFATIERTEN POLYSACCHARID
COMPOSITION COMPRENANT UNE COMBINAISON DE LACTOFERRINE ET UN POLYSACCHARIDE SULFATÉ

(43) Date of publication of application: 18.10.2023
(73) Proprietor: Reckitt Benckiser Health Limited, Slough, Berkshire SL1 3UH (GB)
(72) Inventor: HUSSAIN, Mariam, Slough, SL1 3UH (GB); ROOHPOUR, Nima, Slough, SL1 3UH (GB); SEO, Jin, Montvale, NJ 07645 (US)
(74) Representative: Curran, Clair

(56) References cited:
- WO-A1-2006/001766
- WO-A1-2021/153508
- WO-A1-2021/191904
- CN-A- 112 999 358
- US-A1- 2005 239 742
- WITVROUW M ET AL: "Sulfated polysaccharides extracted from sea algae as potential antiviral drugs", GENERAL PHARMACOLOGY, PERGAMON PRESS, OXFORD, GB, vol. 29, no. 4, 1 January 1997 (1997-01-01), pages 497 - 511, XP002385561, ISSN: 0306-3623

## Description

### Field of the Invention

The present invention relates to a composition for use in the treatment and/or prevention of dysbiosis in a subject, wherein the composition comprises lactoferrin and a carrageenan. In particular, the invention relates to a composition for use in the treatment and/or prevention of dysbiosis of the vaginal microbiota in a subject.

### Background

The microbiota normally associated with the human body have an important influence on human development, physiology, immunity, and nutrition. The vast majority of the indigenous microbiota exist in a mutualistic relationship with their human host, while few are opportunistic pathogens that can cause both chronic infections and life-threatening diseases. These microbial communities are believed to constitute the first line of defence against infection by competitively excluding invasive nonindigenous organisms that cause diseases.

The indigenous microbes within the vagina play a protective role in prevention of colonization of the host by potentially pathogenic organisms, including those responsible for symptomatic bacterial vaginosis, yeast infections, sexually transmitted infections (STI), and urinary tract infections.

*Lactobacilli* species are considered the keystone species of the vaginal microbiota in reproductive-age women of certain ethnicities. The most abundant *Lactobacilli* species are *Lactobacillus iners, L. crispatus,* and *L. gasseri.* Other species as *L. jensenii, L. gallinarum,* and *L. vaginalis* have also been identified in some women.

Several mechanisms underlie the protection exerted by Lactobacilli: competition for nutrients and tissue adherence, reduction of the vaginal pH to -3.5- ~4.5, modulation of immunity, and production of bioactive compounds. Among the bioactive factors of the cervicovaginal mucosa, lactoferrin, an iron-binding cationic glycoprotein, is a multifunctional glycoprotein with antibacterial, antifungal, antiviral, and antiparasitic activities.

When the flora of predominating *Lactobacilli* is reduced in the vaginal environment nonindigenous bacteria may start to colonise.

Chronic or acute disturbances, so called dysbiosis, of the vaginal microbiota may result from the use of antibiotics, fluctuations in hormone levels associated with menstruation, pregnancy, breast feeding and contraceptives, a weakened immune system, sexual activity, age, life style, vaginal lubricants and douching.

Depletion of *Lactobacilli* may predispose to adverse conditions such as preterm birth, acquisition of sexually transmitted infections and pelvic inflammatory disease. Furthermore, depletion of *Lactobacilli* species may lead to bacterial vaginosis (BV), a condition that is characterized by a more diverse vaginal microbiota characterised by *Gardnerella vaginalis, Atopobium vaginae, Megasphaera* and number of other anaerobe or facultative anaerobe bacterial species.

A study by Otsuki *et al.* (2014) demonstrated that the use of vaginal lactoferrin is able to increase the presence of *Lactobacilli* at the level of the vaginal flora demonstrating that the action of the lactoferrin as a prebiotic.

Whilst there have been attempts to develop products that utilise lactoferrin to prevent and/ or treat dysbiosis of the vaginal microbiota, there remains a need for improved compositions that promote the growth of existing commensal vaginal flora, such as strains of vaginal *Lactobaccilli* without promoting the growth of undesirable organisms such as *Gardnerella, Candida* and other opportunistic pathogens.

The plasma membrane of almost all mammalian cells contain negatively charged high molecular weight carbohydrate receptors, such as heparan sulphate glycosaminoglycans. The initial attachment of many different pathogens to host cells involves docking to these carbohydrate receptors. After binding, the pathogens are able to infect the cell by invasion through the cell membranes.

Heparan sulphate proteoglycans (HSPG) are glycoproteins consisting of a core protein linked to one or more linear heparan sulphate (HS) chains. These chains are composed of alternating D-glucosamine and uronic acids (D-glucuronic acid and L-iduronic acids) that can be variably N- and O-sulphated. The presence of sulphate groups at specific positions in HS imparts an overall high negative charge and their arrangement in short segments on the chain create binding sites for HS binding proteins.

HSPGs present a negative charge that can interact electrostatically with HS-binding proteins expressed by the pathogens, for instance with the basic amino acid portions of viral surface proteins. This interaction facilitates the pathogens attachment to host cells, mediates cell entry and increases infectivity. Heparan sulphate glycosaminoglycans can therefore be considered to act as initial, low affinity co-receptors that concentrate pathogens on the cell surface and promote binding to secondary receptors, which stimulates infection.

Disruption of the interaction between the HS on the mammalian cell surface and the HS-binding proteins on the pathogen is therefore a viable therapeutic option for preventing infection.

Some suitable therapeutic options are based on disrupting the binding of pathogenically expressed HS-binding proteins to HS via the competitive binding of positively charged polymers to the HS. Suitable positively charged polymers include carrageenan and chitosan. These polymers rely on electrostatic interactions between their positively charged functional groups and the highly anionic sulphate and carboxylate moieties of HS.

Other compounds that have shown promise as therapeutics are based on the use of exogenous HSPG-mimicking compounds, which function as competitive inhibitors. Examples, include, but are not limited to, sulphated polysaccharides that can block viral infection by physio chemically mimicking the cell surface-bound heparan sulphate, thereby competing against initial virion attachment to the cell surface.

Published PCT application WO2006001766 describes a composition for the treatment of urogenital conditions where it contains lactic acid and lactoferrin and/or a peptide fragment thereof. US patent application no. 11-101,937 describes a method for treating dyspareunia by using a carrageenan in a pharmaceutically acceptable aqueous carrier. A paper published in General Pharmacology, Pergamon Press, Oxford, GB, vol.29, no. 4, 1 January 1997, pages 497-511 details the use of carrageenans as antivirals in the treatment of HIV. Published PCT application WO2021-191904 describes treating viral infections, in particular SARS-CoV-2, using goat milk protein alone or in combination with carrageenan. Chinese Published patent application CN112999358 describes a composition for treating and preventing human papillomavirus using lactoferrin.

There exists a need for compositions suitable for vaginal use that inhibit colonisation of pathogenic microbes whilst respecting the natural commensal vaginal microbiota.

### Summary of Invention

The inventors have surprisingly found that the use of lactoferrin in combination with a carrageenan has a beneficial effect on influencing and maintaining a healthy vaginal microbiota.

According to a first aspect, there is provided a composition according to claim 1.

The carrageenan may be selected from the group consisting of kappa- (κ) carrageenan, iota- (I) carrageenan and lambda- (λ) carrageenan. Preferably, the carrageenan is kappa- (κ) carrageenan.

In some embodiments, the composition comprises lactoferrin in a range of about 5 µg/ml to about 50 µg/ml.

In some embodiments, the composition comprises carrageenan in a range of about 5 µg/ml to about 50 µg/ml.

In some embodiments, the composition comprises lactoferrin in a range of about 10µg/ml to about 30µg/ml and carrageenan in a range of about 10µg/ml to about 30µg/ml.

In some embodiments, the composition is suitable for vaginal administration, and preferably, the composition is a vaginal lubricant and/or vaginal moisturiser.

In some embodiments, the composition is a pharmaceutical composition for use in the treatment of vaginal and cervical-vaginal infections. The infections may be of bacterial, mycotic or viral origin. For example, the infections may be selected from the group consisting of vaginitis, vaginosis, herpes simplex, papilloma virus (HPV), thrush.

According to a second aspect of the invention, there is provided a device for use in the prevention and/or treatment of vaginal and cervical-vaginal infections caused by dysbiosis of the vaginal microbiota, wherein the device comprises a composition according to the first aspect.

In some embodiments, the device is coated or impregnated with the composition according to the first aspect. In some embodiments, the device is a vaginal pessary, a vaginal suppository, a tampon, a contraceptive, a spatula, or an intimate toy.

For example, the device may be a condom that is precoated with a lubricant comprising the composition according to the first aspect.

Non-limiting examples of vaginal and cervical-vaginal inflammations and infections that may be treated using the inventive composition include bacterial vaginosis caused by Gardnerella, chlamydia caused by Chlamydia trachomatis, genital herpes caused by the herpes simplex virus (HSV), genital warts caused by the human papilloma virus (HPV), gonorrhoea, thrush (candidiasis) caused Candida.

### Brief Description of the Figures

Embodiments of the invention will now be described, by way of example only, with reference to the following figures in which:

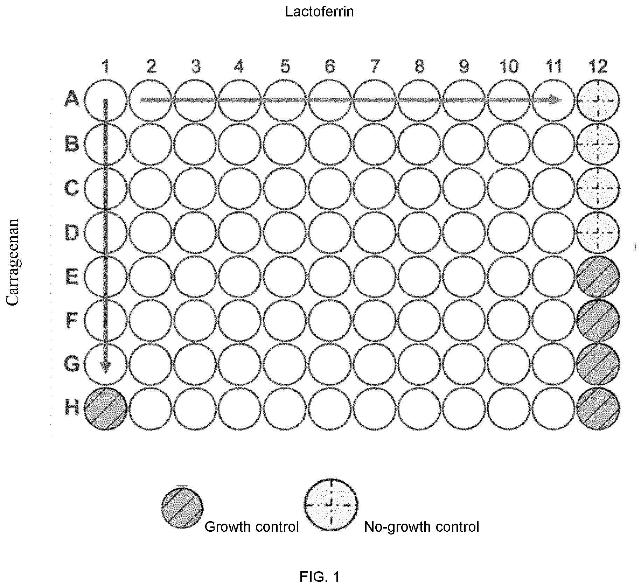

Figure 1 illustrates a checkerboard assay used to assess the combinatorial action of lactoferrin and carrageenan on specific microorganisms;

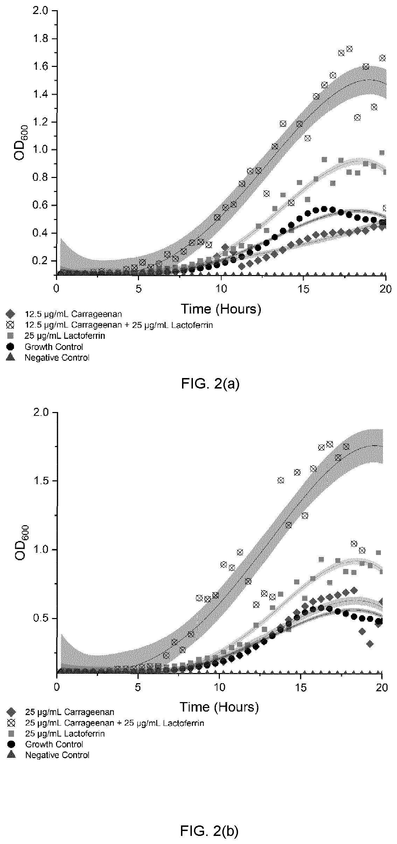

Figure 2 illustrates the prebiotic effect on *Lactobacillus crispatus* 33197 of (a) an exemplary composition of the first aspect containing 12.5 µg/ml κ-carrageenan and 25 µg/ml lactoferrin; and (b) an exemplary composition of the first aspect containing 25 µg/ml κ-carrageenan and 25 µg/ml lactoferrin;

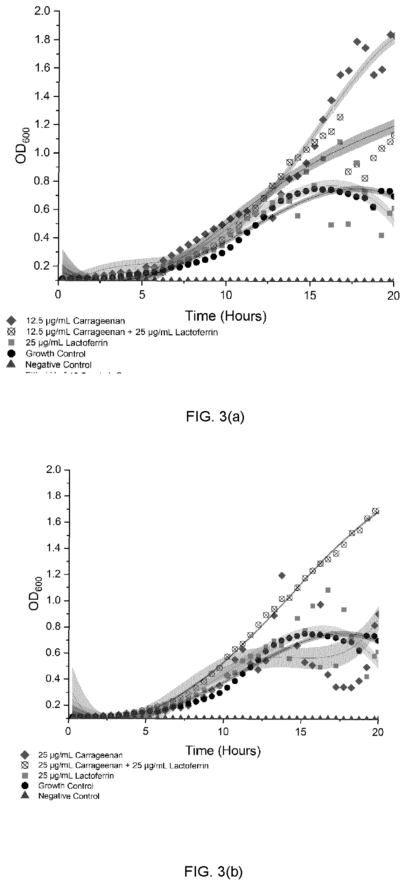

Figure 3 illustrates the prebiotic effect on *Lactobacillus crispatus* 33197 of (a) an exemplary composition of the first aspect containing 12.5 µg/ml I-carrageenan and 25 µg/ml lactoferrin; and (b) an exemplary composition of the first aspect containing 25 µg/ml I-carrageenan and 25 µg/ml lactoferrin;

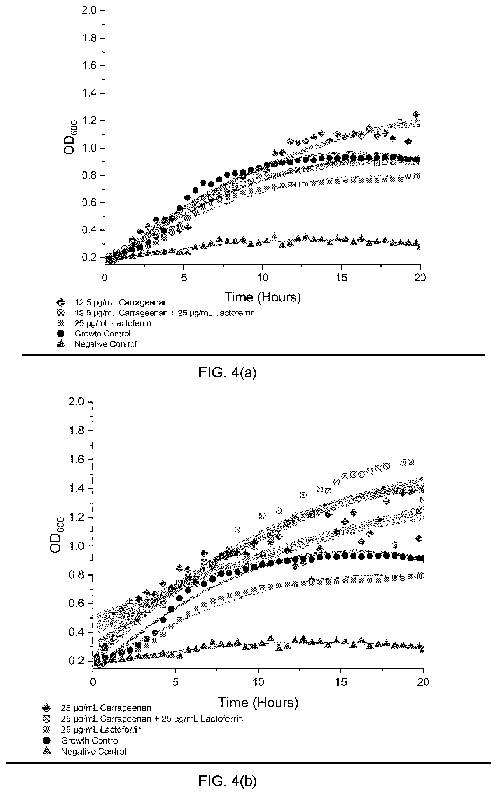

Figure 4 illustrates the prebiotic effect on *Lactobacillus jensenii 25258* of (a) an exemplary composition of the first aspect containing 12.5 µg/ml κ-carrageenan and 25 µg/ml lactoferrin; and (b) an exemplary composition of the first aspect containing 25 µg/ml κ -carrageenan and 25 µg/ml lactoferrin;

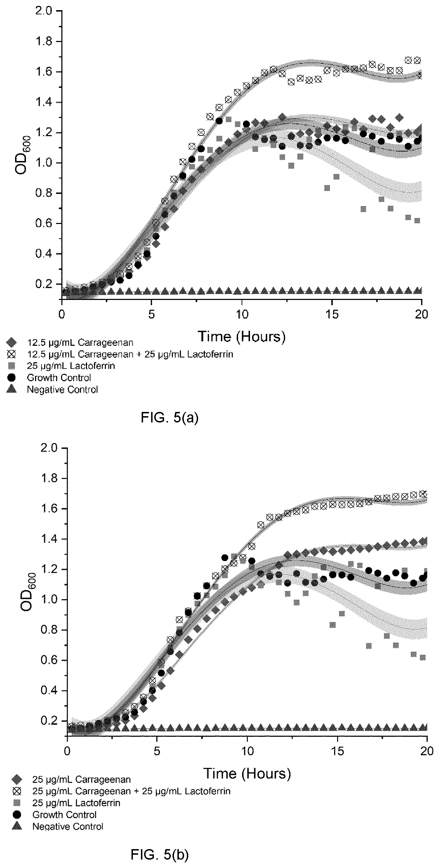

Figure 5 illustrates the prebiotic effect on *Lactobacillus jensenii 25258* of (a) an exemplary composition of the first aspect containing 12.5 µg/ml I-carrageenan and 25 µg/ml lactoferrin; and (b) an exemplary composition of the first aspect containing 25 µg/ml I-carrageenan and 25 µg/ml lactoferrin;

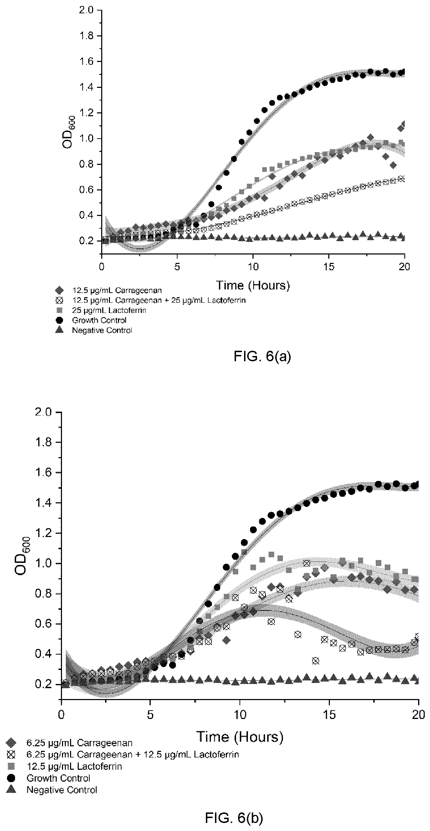

Figure 6 illustrates the antimicrobial effect against *Gardenia vaginalis 14018* of (a) an exemplary composition of the first aspect containing 12.5 µg/ml κ-carrageenan and 25 µg/ml lactoferrin and (b) an exemplary composition of the first aspect containing 6.25 µg/ml κ-carrageenan and 12.5 µg/ml lactoferrin.

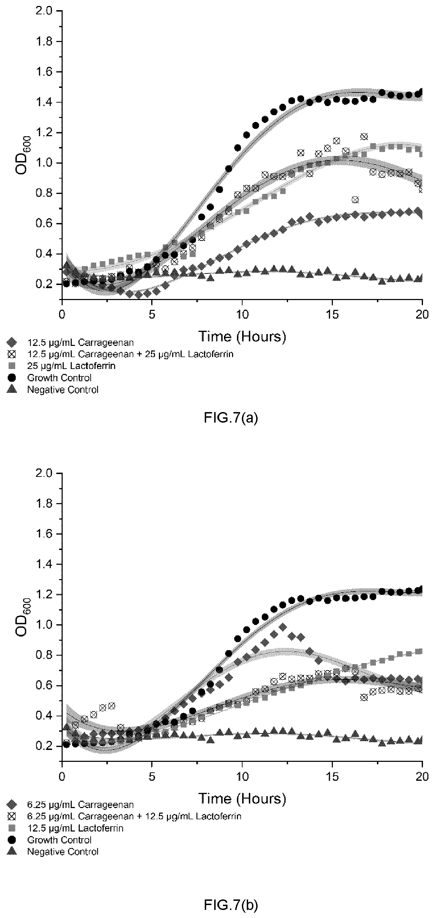

Figure 7 illustrates the antimicrobial effect against *Gardenia vaginalis 14018* of (a) an exemplary composition of the first aspect containing 12.5 µg/ml I-carrageenan and 25 µg/ml lactoferrin; and (b) an exemplary composition of the first aspect containing 6.25 µg/ml I-carrageenan and 12.5 µg/ml lactoferrin.

### Definitions

"Dysbiosis" or "dysbiotic state" refers to an imbalance between the types of organism present in a person's natural microflora, thought to contribute to a range of conditions of ill health.

"Eubiosis" or "eubiotic state" refers to an optimal balance between the types of organism present in a person's natural microflora, thought to contribute to a range of conditions of ill health.

"Non-human lactoferrin" refers to lactoferrin that is produced by or obtained from a source other than human breast milk, for example bovine lactoferrin.

"Personal lubricant(s)" refers to specialized lubricants used during intimate acts, such as intercourse and masturbation, to reduce friction to or between the penis and vagina, anus or other body parts or applied to sex toys to reduce friction or to ease penetration. These differ from surgical or medical lubricants or gels, which are similar to personal lubricants which may be used for medical purposes such as speculum insertion.

"Reconstituted solution" refers to a solution prepared when a diluent (e g. water, saline, etc.) is added to an ingredient (e g. a powder, a solution, a gel, a suspension, a paste, a solid, a liquid, a liquid concentrate, etc.). When the term "reconstituted solution" is used in reference to lactoferrin & carrageenan, this means the "solution prepared by the addition of a diluent (e.g., water, saline, etc.) to a form of lactoferrin/lactoferrin-containing/ carrageenan, carrageenan-containing composition i.e., in the form of a powder, a solution, a gel, a suspension, a paste, a solid, a liquid, a liquid concentrate, etc.

All percentages, parts, and ratios as used herein are detailed by weight of the total formulation, unless otherwise specified. All amounts specified as administered "per day" may be delivered in a single unit dose, in a single serving, or in two or more doses or servings administered over the course of a 24-hour period.

All references to singular characteristics or limitations in the present disclosure shall include the corresponding plural characteristic or limitation, and vice versa, unless otherwise specified or clearly implied to the contrary, by the context in which the reference is made.

All combinations of method or process steps disclosed herein can be performed in any order, unless otherwise specified or clearly implied to the contrary, by the context in which the referenced combination is made.

The formulations of the present disclosure can comprise, consist of, or consist essentially of any of the components described herein, as well as including any additional useful component.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

### Detailed Description

Lactoferrin is a polypeptide belonging to the family of transferrins. In nature, lactoferrin is present in many mucous secretions, including vaginal secretions. The concentration of lactoferrin in human vaginal fluid corresponds to 1~3 µg/ml (Valenti P, 2018) while it is extremely high (100 µg/ml) in the cervical mucus plug (Heim, M, 2002)

Lactoferrin's antimicrobial activity has been indicated to be effective toward bacteria, viruses, fungi, and parasites (Farnaud S, 2003).

The antimicrobial activity of lactoferrin is linked to its affinity for ferric ion (Fe³⁺). The combination of lactoferrin with the ferric ion in mucous secretions modulates the proliferation and aggregation of bacteria and of viruses towards the cellular membranes.

The lactoferrin of any aspect detailed herein may comprise human lactoferrin produced by a genetically modified organism, humanised lactoferrin, non-human lactoferrin, or any combination thereof. The non-human lactoferrin may comprise bovine lactoferrin, porcine lactoferrin, equine lactoferrin, buffalo lactoferrin, goat lactoferrin, murine lactoferrin, or camel lactoferrin.

Carrageenan is a generic term for linear galactose-based polysaccharides extracted from seaweed (Rhodophyceae). Carrageenans are made up of repeating units of sulphated D-galactose and 3,6-anhydro-D-galactose. There exist more than 10 structurally different carrageenans, their nature depending on the seaweed genus from which they are extracted. The three main types are carrageenan, which differ in their degree of sulfation are kappa (κ) carrageenan has one sulphate group per disaccharide, iota (I) carrageenan has two sulphates and lambda (λ) carrageenan has three.

The food, cosmetic, and pharmaceutical industries use carrageenans extensively as thickening, gelling and stabilising agents. They are classified as "generally recognised as safe" (GRAS) by the Food and Drug Administration (FDA). In addition to its advantageous physical properties, carrageenan also demonstrates antiviral activity, against a range of viruses including human papillomaviruses (HPV).

In addition to interacting with many different virus particles and bacteria, carrageenan may also form a mucoadhesive layer on the surface of the vaginal epithelium, thereby forming a shielding layer on their surfaces preventing interaction with the epithelium and infection of the cells.

The advantageous combination of carrageenan's low coefficient of friction and its antimicrobial properties has led to its inclusion in some brands of vaginal lubricants and spermicides.

The carrageenans useful in the present invention are commercially available but may also be prepared by extraction from seaweed plants pursuant to extraction procedures known in the art.

The carrageenan of the present invention may be a homopolymer or a heteropolymer. A carrageenan homopolymer is built of subunits of only one kind of κ, I or λ carrageenan, wherein a heteropolymer comprises subunits of either of two or more of the carrageenans.

The composition may comprise lactoferrin in the range of about 5 µg/ml to about 50 µg/ml. In some embodiments the composition may comprise lactoferrin in the range of 10 µg/ml to about 40 µg/ml, 15 µg/ml to about 35 µg/ml, or about 20 µg/ml to about 25 µg/ml.

In some particular embodiments, the composition comprises lactoferrin in combination with a x-carrageenan.

The lactoferrin may be present in an amount ranging from about 5 µg/ml to about 50 µg/ml. In some embodiments the composition may comprise lactoferrin in the range of 10 µg/ml to about 40 µg/ml, 15 µg/ml to about 35 µg/ml, or about 20 µg/ml to about 25 µg/ml. The κ-carrageenan may be present in an amount ranging from about 5 µg/ml to about 50 µg/ml. In some embodiments the composition may comprise K -carrageenan in the range of 10 µg/ml to about 40 µg/ml, 15 µg/ml to about 35 µg/ml, or about 20 µg/ml to about 25 µg/ml.

In some particular embodiments, the composition comprises lactoferrin in combination with a I -carrageenan.

The lactoferrin may be present in an amount ranging from about 5 µg/ml to about 50 µg/ml. In some embodiments the composition may comprise lactoferrin in the range of 10 µg/ml to about 40 µg/ml, 15 µg/ml to about 35 µg/ml, or about 20 µg/ml to about 25 µg/ml. The I-carrageenan may be present in an amount ranging from about 5 µg/ml to about 50 µg/ml. In some embodiments the composition may comprise I-carrageenan in the range of 10 µg/ml to about 40 µg/ml, 15 µg/ml to about 35 µg/ml, or about 20 µg/ml to about 25 µg/ml.

In some particular embodiments, the composition comprises lactoferrin, a x-carrageenan and a I-carrageenan. Advantageously, such a composition acts as a broad spectrum prebiotic. For example, a combination of lactoferrin with κ-carrageenan has been shown to demonstrate a prebiotic effect for *Lactobacillus crispatus,* whereas a I-carrageenan has been shown to demonstrate a prebiotic effect for *Lactobacillus jensenii.*

The lactoferrin may be present in an amount ranging from about 5 µg/ml to about 50 µg/ml. In some embodiments the composition may comprise lactoferrin in the range of 10 µg/ml to about 40 µg/ml, 15 µg/ml to about 35 µg/ml, or about 20 µg/ml to about 25 µg/ml. The x-carrageenan may be present in an amount ranging from about 5 µg/ml to about 50 µg/ml. In some embodiments the composition may comprise κ-carrageenan in the range of 10 µg/ml to about 40 µg/ml, 15 µg/ml to about 35 µg/ml, or about 20 µg/ml to about 25 µg/ml. The I-carrageenan may be present in an amount ranging from about 5 µg/ml to about 50 µg/ml. In some embodiments the composition may comprise I-carrageenan in the range of 10 µg/ml to about 40 µg/ml, 15 µg/ml to about 35 µg/ml, or about 20 µg/ml to about 25 µg/ml

In some particular embodiments, the composition comprises lactoferrin in a range of about 10-30 µg/ml and κ-carrageenan in a range of about 10-30 µg/ml.

In some particular embodiments, the composition comprises about 25 µg/ml lactoferrin and about 12.5 µg/ml x-carrageenan.

In some particular embodiments, the composition comprises about 25 µg/ml lactoferrin and 25 µg/ml x-carrageenan.

In some particular embodiments, the composition comprises lactoferrin in a range of about 10-30 µg/ml and I-carrageenan in a range of about 10-30 µg/ml.

In some particular embodiments, the composition comprises about 25 µg/ml lactoferrin and about 12.5 µg/ml I-carrageenan.

In some particular embodiments, the composition comprises about 25 µg/ml lactoferrin and 25 µg/ml I-carrageenan.

In some particular embodiments, the composition comprises lactoferrin in a range of about 10-30 µg/ml, κ-carrageenan in a range of about 10-30 µg/ml, and I-carrageenan in a range of about 10-30 µg/ml.

In some particular embodiments, the composition comprises about 25 µg/ml lactoferrin, about 12.5µg/ml κ-carrageenan and about 12.5 µg/ml I-carrageenan.

In some particular embodiments, the composition comprises about 25 µg/ml lactoferrin, about 25µg/ml κ-carrageenan and about 25 µg/ml I-carrageenan.

The composition according to the first aspect may be administered to the subject by topical intravaginal administration.

The composition according to the first aspect may be in the form of a powder, a solution, a gel, a suspension, a paste, a solid, a liquid, a liquid concentrate, or a reconstituted solution. In some particular embodiments, the composition is administered to the subject in the form of a personal lubricant.

According to a second aspect of the invention, there is provided a device comprising the composition of the first aspect. In some embodiments, the composition is coated or impregnated on a device that is inserted into the vagina. The device may be permanently or transiently inserted into the vagina. For example, the device may be a vaginal pessary, vaginal suppository, a tampon, a contraceptive, a spatula, or an intimate toy. The contraceptive may be a condom. The condom may be a male and/or female condom.

In some embodiments, the composition is attached to a surface of the device, for example by physical and/or chemical attachment. In some other embodiments, the device is lubricated with the composition.

The composition as described herein may be used prophylactically by a subject to maintain a eubiotic vaginal microbiota. Hence, the composition according to the first aspect may be used non-therapeutically.

The composition may be used therapeutically by a subject to treat dysbiosis of the vaginal microbiota in an attempt push the microbiota towards a eubiotic state.

In an unclaimed embodiment, there is provided a method for the prevention and/ or treatment of vaginal dysbiosis in a subject, the method comprising the step of administering to a subject a composition according to the first aspect. More particularly, the method may be used in the prevention and/or treatment of vaginal and/or cervical-vaginal infections.

In some unclaimed embodiments, the method may include administering the composition to a subject, at a sufficient frequency and for a sufficient period of time, to promote the growth of at least some species of vaginal *Lactobacilli* whilst reducing or preventing the growth of pathogenic species. As such, the composition strengthens the native *Lactobacilli* population, thereby promoting an environment hostile to vaginal pathogens.

More specifically, the unclaimed method may include administering the composition to a subject at a sufficient frequency and for a sufficient period of time to promote the growth of *Lactobacillus crispatus* and/or *Lactobacillus jensenii* whilst reducing or preventing the growth *Gardneralla vaginalis* and/or *Candida albicans.*

The frequency of administration of the composition to the subject may be varied depending on the requirement of the subject. The composition may, for example, be administered daily.

The composition may also contain a probiotic. In particular, the probiotic may be selected among *Lactobacilli, bifidobacteria pediococci* and mixtures thereof. Preferably as *Lactobacilli* at least one of the following is used: *Lactobacillus plantarum, Lactobacillus bulgaricus, Lactobacillus casei, Lactobacillus bulgaricus, Lactobacillus paracasei, Lactobacillus rhamnosus, Lactobacillus acidophilus, Lactobacillus fermentum, Lactobacillus gasseri, Lactobacillus crispatus, Lactobacillus iners, Lactobacillus jensenii, Lactobacillus gallinarum,* and *Lactobacillus. vaginalis* and as *Bifidobacteria* at least one of the following is used: *Bifidobacterium lactis, Bifidobacterium bifidum,* and mixtures thereof.

### EXAMPLES

### Method

A stock solution of 8 mg/mL bovine lactoferrin, was prepared by adding the appropriate amount of sterile MilliQ water to the weighted compound, followed by vortexing and filter-sterilizing.

A stock solution of 2 mg/mL of each of the carrageenans (κ-,I-,λ-) was prepared by adding the appropriate amount of sterile MilliQ water to the weighted compound, incubated at 37°C for 30 minutes, followed by vortexing until clear, and filter-sterilizing.

As shown in Figure 1, a 96-well microtiter plate chequerboard assay was utilized to evaluate the synergistic combinatorial effect of lactoferrin and/or carrageenan (κ-,I-,λ-) at various doses against a particular microorganism. The use of the chequerboard assay enabled an assessment of whether a specific ingredient combination possessed a prebiotic or antimicrobial effect.

The stock solution of lactoferrin was serially diluted in a broth-based media (appropriate for growth of the particular microorganism to be assayed) along the rows of the microtiter plate.

The stock solution of carrageenan (κ-,I-, or A-) was serially diluted in a broth-based media (appropriate for growth of the particular microorganism) along the columns of the microtiter plate.

The final volume of media in each well was 0.1 mL.

Each well containing lactoferrin and/or carrageenan (κ-, I-, or A-) was inoculated with log-phase bacteria/yeast at a concentration of 5 x 10⁵ colony forming units (CFU)/well.

The positive (growth) control consisted of the relevant microorganism seeded in a broth-based media.

The negative (no-growth) control consisted of a broth-based media without any microorganism present.

A summary of the Examples is presented below in Table 1.

**TABLE 1**

| Example | FIG. | Microorganism | Lactoferrin [µg/ml] | Carrageenan class | Carrageenan [µg/ml] |
|---|---|---|---|---|---|
| 1 | 2a | *Lactobacillus crispatus* 33197 | 25 | kappa | 12.5 |
| | 2b | *Lactobacillus crispatus* 33197 | 25 | kappa | 25 |
| 2 | 3a | *Lactobacillus crispatus* 33197 | 25 | iota | 12.5 |
| | 3b | *Lactobacillus crispatus* 33197 | 25 | iota | 25 |
| 3 | 4a | *Lactobacillus jensenii 25258* | 25 | kappa | 12.5 |
| | 4b | *Lactobacillus jensenii 25258* | 25 | kappa | 25 |
| 4 | 5a | *Lactobacillus jensenii 25258* | 25 | iota | 12.5 |
| | 5b | *Lactobacillus jensenii 25258* | 25 | iota | 25 |
| 5 | 6a | *Gardenia vaginalis 14018* | 25 | kappa | 12.5 |
| | 6b | *Gardenia vaginalis 14018* | 12.5 | kappa | 6.25 |
| 6 | 7a | *Gardenia vaginalis 14018* | 25 | iota | 12.5 |
| | 7b | *Gardenia vaginalis 14018* | 12.5 | iota | 6.25 |

After addition of the relevant microorganism, plates were sealed with optical quality film, and incubated within the plate reader at the appropriate growth temperature for the microorganism being assayed. The optical density (OD) was recorded every 15 minutes for a period of 48 hours to evaluate the microorganism growth and biomass formation.

### Results

### PREBIOTIC EFFECT OF A COMPOSITION COMPRISING LACTOFERRIN AND CARRAGEENAN

Examples 1-4 demonstrate the prebiotic effect of a combination of lactoferrin with different classes of carrageenan on either *Lactobacillus crispatus* 33197 and *Lactobacillus jensenii 25258.*

### Example 1: Effect of a combination of κ-carrageenan and lactoferrin on L.crispatus 33197

The prebiotic effect of lactoferrin and κ-carrageenan alone and in combination at different concentrations was assessed against *L.crispatus 33197*. *L.crispatus* is commensal bacteria within the vaginal microbiota, and therefore a composition that has a prebiotic effect on this bacterium is particularly advantageous.

Figure 2a shows the effect of 12.5 µg/ml κ-carrageenan and/or 25 µg/ml lactoferrin and Figure 2b shows the effect of 25 µg/ml κ-carrageenan and 25 µg/ml lactoferrin.

A combination of 25 µg/ml lactoferrin with either 12.5 µg/ml (FIG. 2a) or 25 µg/ml (FIG. 2b) of κ-carrageenan demonstrated a synergistic effect on the growth of *L.crispatus* 33197. The combination has a prebiotic effect, not only increasing the growth but also reducing the initial lag phase. The lag phase is one of the earliest phases of the bacterial growth cycle, and is the phase in which the bacterium tends to adapt itself to the growth conditions; maturing but not dividing.

### Example 2: Effect of a combination of I-carrageenan and lactoferrin on L.crispatus 33197

The prebiotic effect of lactoferrin and I-carrageenan alone and in combination at different concentrations was assessed against *L.crispatus 33197*. *L.crispatus* is commensal bacteria within the vaginal microbiota, and therefore a composition that has a prebiotic effect on this bacterium is particularly advantageous.

Figure 3a shows the effect of 12.5 µg/ml I -carrageenan and/or 25 µg/ml lactoferrin and Figure 3b shows the effect of 25 µg/ml I -carrageenan and 25 µg/ml lactoferrin.

A combination of 25 µg/ml lactoferrin with 25 µg/ml of I-carrageenan (FIG. 3b) has a synergistic effect on the growth of *L.crispatus 33197*, which becomes pronounced at around 10 hrs. The composition has a prebiotic effect, not only increasing the growth but also reducing the lag phase.

### Example 3: Effect of a combination of κ-carrageenan and lactoferrin on L. jensenii 25258

The prebiotic effect of lactoferrin and κ-carrageenan alone and in combination at different concentrations was assessed on *L. jensenii 25258. L. jensenii* is commensal bacteria within the vaginal microbiota, and therefore a composition that has a prebiotic effect on this bacterium is particularly advantageous.

Figure 4a shows the effect of 12.5 µg/ml κ-carrageenan and/or 25 µg/ml lactoferrin and Figure 4b shows the effect of 25 µg/ml κ -carrageenan and 25 µg/ml lactoferrin.

As shown in FIG. 4a, a combination of 25 µg/ml lactoferrin with 12.5 µg/ml of κ-carrageenan has less of an impact on the growth of *L. jensenii 25258* than when 12.5 µg/ml of κ-carrageenan is tested alone. However, as shown in FIG. 4b, an improvement in the prebiotic effect is observed for a combination of 25 µg/ml lactoferrin with 25 µg/ml of x-carrageenan.

### Example 4: Effect of a combination of I-carrageenan and lactoferrin on L. jensenii 25258

The prebiotic effect of lactoferrin and I-carrageenan alone and in combination with each other at different concentrations was assessed on *L. jensenii 25258. L. jensenii* is commensal bacteria within the vaginal microbiota, and therefore a composition that has a prebiotic effect on this bacterium is particularly advantageous.

Figure 5a shows the effect of 25 µg/ml lactoferrin with 12.5 µg/ml I-carrageenan and Figure 5b shows the effect of 25 µg/ml lactoferrin with 25 µg/ml I-carrageenan.

As shown in FIG. 5, a composition comprising a combination of 25µg/ml lactoferrin with 12.5 µg/ml I-carrageenan (FIG. 5a) or 25 µg/ml lactoferrin with 25µg/ml I-carrageenan (FIG. 5b) has a synergistic effect on the growth of *L. jensenii 25258.* The combination maintains the viability of *L. jensenii 25258* in the stationary phase of the bacterial growth cycle, whereas in comparison when the bacteria are exposed to lactoferrin or I-carrageenan alone, the bacteria enters the death phase.

### ANTIMICROBIAL EFFECT OF A COMPOSITION COMPRISING LACTOFERRIN AND CARRAGEENAN

Examples 5 and 6 demonstrate the antimicrobial effect of a combination of lactoferrin with different classes of carrageenan on *Gardenia* vaginalis *14018. Garderella vaginallis* is a bacterium which can cause bacterial vaginosis (BV), and therefore a composition that has an antimicrobial effect against this bacteri is particularly advantageous.

### Example 5: Effect of a combination of κ-carrageenan and lactoferrin on Gardenia vaginalis 14018

The antimicrobial effect of lactoferrin and x-carrageenan alone and in combination at different concentrations was assessed against *Gardenia* vaginalis *14018.*

Figure 6a shows the effect of 25 µg/ml lactoferrin with 12.5 µg/ml κ-carrageenan and Figure 6b shows the effect of or 12.5 µg/ml lactoferrin with 6.25 µg/ml κ-carrageenan.

As shown in FIG. 6 a composition comprising a combination of 25 µg/ml lactoferrin with either 12.5 µg/ml κ-carrageenan (FIG. 6a) or 12.5 µg/ml lactoferrin with 6.25 µg/ml κ-carrageenan (FIG. 6b) has a synergistic effect on the inhibition of growth of *Gardenia* vaginalis *14018.*

### Example 6: Effect of a combination of I-carrageenan and lactoferrin on Gardenia vaginalis 14018

The antimicrobial effect of lactoferrin and I-carrageenan alone and in combination at different concentrations was assessed against on *Gardenia* vaginalis *14018.*

Figure 7a shows the effect of 25 µg/ml lactoferrin with 12.5 µg/ml I-carrageenan and Figure 7b shows the effect of or 12.5 µg/ml lactoferrin with 6.25 µg/ml I-carrageenan.

As shown in FIG. 7 a composition comprising a combination of 25 µg/ml lactoferrin with 12.5 µg/ml I-carrageenan (FIG. 7a) or 12.5 µg/ml lactoferrin with 6.25 µg/ml (FIG. 7b) I-carrageenan inhibits the growth of *Gardenia* vaginalis *14018* when compared to the positive control, but only at comparative level to lactoferrin or I-carrageenan when taken alone.

### CONCLUSION

It is desirable for a composition for vaginal administration, such as a personal lubricant, to be able to prevent and/or treat dysbiosis of the vaginal microbiota by promoting the growth of commensal bacteria whilst inhibiting the growth of pathogenic bacteria.

The Examples above demonstrate that, in particular, a composition comprising a combination of lactoferrin and x-carrageenan is particularly advantageous, not only demonstrating a synergistic prebiotic effect against *L.crispatus 33197* (see Example 1) but also a synergistic antimicrobial effect against Gardenia vaginalis 14018 (see Example 5).

A composition comprising a combination of lactoferrin and I-carrageenan is advantageous, demonstrating a synergistic prebiotic effect against *L.crispatus 33197* (see Example 2) but demonstrating an antimicrobial effect (comparable to lactoferrin or I-carrageenan alone) against *Gardenia vaginalis* 14018 (see Example 6).

### REFERENCES

Farnaud, S. and Evans, R.W. Lactoferrin: a multifunctional protein with antimicrobial properties. Molecular Immunology. 2003; 40(7):395-405.
Hein M. et al. Antimicrobial factors in the cervical mucus plug. Am. J. Obstet. Gynecol. 2002 Jul;187(1):137-44.
Valenti P. et al. Role of lactobacilli and lactoferrin in the mucosal cervicovaginal defense. Front Immunol. 2018; 9:376.
Otsuki K. et al. Administration of oral and vaginal prebiotic lactoferrin for a woman with refractory vaginitis recurring preterm delivery: Appearance of lactobacillus in vaginal flora followed by term delivery. The Journal of Obstet. Gynaecol. Res. 2014 Feb; 40(2):585-585.

## Claims

1. A composition comprising lactoferrin and a carrageenan for use in the treatment and/or prevention of vaginal dysbiosis in a subject.

2. The composition for use according to claim 1, wherein the carrageenan is selected from the group consisting of kappa- (κ) carrageenan, iota- (I) carrageenan and lambda- (λ) carrageenan.

3. The composition for use according to claim 1 or 2, wherein the composition comprises lactoferrin in a range of about 5 µg/ml to about 50 µg/ml.

4. The composition for use according to any preceding claim, wherein the composition comprises a carrageenan in a range of about 5 µg/ml to about 50 µg/ml.

5. The composition for use according to any preceding claim, wherein the composition comprises lactoferrin in a range of about 10 µg/ml to about 30 µg/ml and a carrageenan in a range of about 10 µg/mlto about 30 µg/ml.

6. The composition for use according to any preceding claim, wherein the composition is suitable for vaginal administration, and preferably wherein the composition is a personal lubricant.

7. The composition for use according to any preceding claim, wherein the composition is a pharmaceutical composition.

8. The composition for use according to any preceding claim, wherein the dysbiosis leads to vaginal and cervical-vaginal infections.

9. The composition for use according to claim 8, wherein the infections are of bacterial, mycotic or viral origin.

10. The composition for use according to claim 9, wherein the infections are selected from the group consisting of vaginitis, vaginosis, herpes simplex, papilloma virus (HPV), thrush.

11. The composition for use according to any preceding claim, wherein the composition coats or impregnates a device.

12. The composition for use according to claim 11, wherein the device is a vaginal pessary, vaginal suppository, a tampon, a contraceptive, a spatula, or an intimate toy.

## Patentansprüche

1. Zusammensetzung, umfassend Laktoferrin und Carrageenan zur Verwendung bei der Behandlung und/oder Prävention von Vaginaldysbiose bei einem Subjekt.

2. Zusammensetzung zur Verwendung nach Anspruch 1, wobei das Carrageenan ausgewählt ist aus der Gruppe bestehend aus Kappa (κ) -Carrageenan, Iota ( 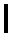 ) - Carrageenan und Lambda (λ) -Carrageenan.

3. Zusammensetzung zur Verwendung nach Anspruch 1 oder 2, wobei die Zusammensetzung Laktoferrin in einem Bereich von etwa 5 µg/ml bis etwa 50 µg/ml umfasst.

4. Zusammensetzung zur Verwendung nach einem der vorhergehenden Ansprüche, wobei die Zusammensetzung ein Carrageenan in einem Bereich von etwa 5 µg/ml bis etwa 50 µg/ml umfasst.

5. Zusammensetzung zur Verwendung nach einem der vorhergehenden Ansprüche, wobei die Zusammensetzung Laktoferrin in einem Bereich von etwa 10 µg/ml bis etwa 30 µg/ml und ein Carrageenan in einem Bereich von etwa 10 µg/ml bis etwa 30 µg/ml umfasst.

6. Zusammensetzung zur Verwendung nach einem der vorhergehenden Ansprüche, wobei die Zusammensetzung zur vaginalen Verabreichung geeignet ist und wobei bevorzugt die Zusammensetzung ein Gleitmittel ist.

7. Zusammensetzung zur Verwendung nach einem der vorhergehenden Ansprüche, wobei die Zusammensetzung eine pharmazeutische Zusammensetzung ist.

8. Zusammensetzung zur Verwendung nach einem der vorhergehenden Ansprüche, wobei die Dysbiose zu vaginalen und zervikal-vaginalen Infektionen führt.

9. Zusammensetzung zur Verwendung nach Anspruch 8, wobei die Infektionen bakteriellen, mykotischen oder viralen Ursprungs sind.

10. Zusammensetzung zur Verwendung nach Anspruch 9, wobei die Infektionen ausgewählt sind aus der Gruppe bestehend aus Vaginitis, Vaginose, Herpes simplex, Papillomavirus (HPV), Soor.

11. Zusammensetzung zur Verwendung nach einem der vorhergehenden Ansprüche, wobei die Zusammensetzung eine Vorrichtung überzieht oder imprägniert.

12. Zusammensetzung zur Verwendung nach Anspruch 11, wobei die Vorrichtung ein Vaginalpessar, ein Vaginalzäpfchen, ein Tampon, ein Verhütungsmittel, eine Spatel oder ein Intimspielzeug ist.

## Revendications

1. Composition comprenant de la lactoferrine et un carraghénane pour utilisation dans le traitement et/ou la prévention de la dysbiose vaginale chez un sujet.

2. Composition pour utilisation selon la revendication 1, dans laquelle le carraghénane est choisi dans le groupe consistant en kappa-(κ) carraghénane, iota-( 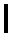 ) carraghénane et lambda-(λ) carraghénane.

3. Composition pour utilisation selon la revendication 1 ou 2, dans laquelle la composition comprend de la lactoferrine dans une plage d'environ 5 µg/ml à environ 50 µg/ml.

4. Composition pour utilisation selon l'une quelconque des revendications précédentes, dans laquelle la composition comprend un carraghénane dans une plage d'environ 5 µg/ml à environ 50 µg/ml.

5. Composition pour utilisation selon l'une quelconque des revendications précédentes, dans laquelle la composition comprend de la lactoferrine dans une plage d'environ 10 µg/ml à environ 30 µg/ml et un carraghénane dans une plage d'environ 10 µg/ml à environ 30 µg/ml.

6. Composition pour utilisation selon l'une quelconque des revendications précédentes, dans laquelle la composition est adaptée à une administration vaginale, et de préférence dans laquelle la composition est un lubrifiant personnel.

7. Composition pour utilisation selon l'une quelconque des revendications précédentes, dans laquelle la composition est une composition pharmaceutique.

8. Composition pour utilisation selon l'une quelconque des revendications précédentes, dans laquelle la dysbiose conduit à des infections vaginales et cervico-vaginales.

9. Composition pour utilisation selon la revendication 8, dans laquelle les infections sont d'origine bactérienne, mycotique ou virale.

10. Composition pour utilisation selon la revendication 9, dans laquelle les infections sont choisies dans le groupe consistant en vaginite, vaginose, herpès simplex, papillomavirus (HPV), muguet.

11. Composition pour utilisation selon l'une quelconque des revendications précédentes, dans laquelle la composition recouvre ou imprègne un dispositif.

12. Composition pour utilisation selon la revendication 11, dans laquelle le dispositif est un pessaire vaginal, un suppositoire vaginal, un tampon, un contraceptif, une spatule ou un jouet intime.
